# EUROPEAN PATENT APPLICATION

(11) **EP 2 053 040 A1**
(43) Date of publication of application: **29.04.2009**
(21) Application number: 07119355.1
(22) Date of filing: 26.10.2007
(51) Int. Cl.: C07C 253/30, C07D 207/12, C07D 207/26, A61K 31/197

(54) **Pregabalin intermediates and process for preparing them and Pregabalin**

(71) Applicant: Chemo Ibérica, S.A., 08028 Barcelona (ES)
(72) Inventor: Rasparini, Marcello, 27010, Cura Carpignano (PV) (IT); Tufaro, Roberto, 28060, Sozzago (NO) (IT); Marras, Giovanni, 28100, Novara (IT); Giovenzana, Giovanni, 28100, Novara (IT); Castaldi, Graziano, 28072, Briona (NO) (IT)
(74) Representative: Barlocci, Anna

(57) **Abstract**

The present invention provides novel compounds, which are especially useful as intermediates for the preparation of Pregabalin. The invention also relates to process for preparing these intermediates and Pregabalin.

## Description

### Field of the invention

The present invention relates to novel compounds, which are especially useful as intermediates for the preparation of Pregabalin. The invention also relates to a process for preparing these intermediates and Pregabalin.

### Background of the invention

(±)-3-Aminomethyl-5-methyl-1-hexanoic acid (Pregabalin), which is also called β-isobutyl-γ-aminobutyric acid or isobutyl-GABA, is related to the endogenous inhibitory neurotransmitter γ-aminobutyric acid (GABA). GABA is one of the most widely distributed inhibitory neurotransmitters involved in the regulation of brain neuronal activity. The concentration of GABA is regulated by two pyridoxal 5'-phosphate dependent enzymes: L-glutamic acid decarboxylase (GAD), which catalyzes conversion of L-glutamic acid to GABA, and GABA aminotransferase, which degrades GABA to succinic semialdehyde. When the concentration of GABA diminishes below a threshold level in the brain, convulsions may result and, conversely, raising the GABA level appears to terminate seizures. The term "seizure" means excessive unsynchronized neuronal activity that disrupts normal brain function.
Pregabalin increases the concentration of GABA by activating GAD and is a potent anticonvulsant: it has the ability to suppress seizure while avoiding the undesirable side effect of ataxia. Pregabalin is useful as a therapeutic agent for the treatment of pain, epilepsy, convulsions, psychiatric disorders, attention deficit, hypersensitivity disorder, anxiety and mood disorders.

It has been discovered that the anticonvulsant effect of Pregabalin is dependent on its stereochemistry. The anticonvulsant effect of racemic Pregabalin is primarily attributable to the (S)-Pregabalin. (S)-Pregabalin shows better anticonvulsant activity, than the (R)-stereoisomer (see, for example, Yuen et al., Bioorganic & Medicinal Chemistry Letters, 1994, 4, 823).
(S)-Pregabalin is a compound of Formula (I) 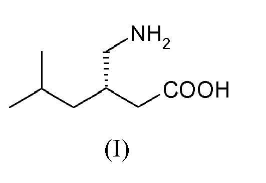 [(S)-(+)-3-aminomethyl-5-methyl-1-hexanoic acid], which is disclosed in EP 641 330 B1 and marketed under the trade name Lyrica^{®}.

The commercial utility of Pregabalin requires an efficient, cost effective and safe method for preparing the S-enantiomer substantially free of the R-enantiomer in a large scale.

Several methods have been used to prepare (S)-Pregabalin, *via* diastereomeric salt resolution or *via* an asymmetric synthesis.

In a typical resolution process, the racemic mixture is converted to a mixture of diastereomeric salts by reaction with a suitable chiral non racemic resolving agent, which is tipically a chiral acid or base, often selected from naturally occurring compounds, in a suitable solvent or solvent mixture. Unlike the enantiomers from which they are formed, the diastereomers frequently have very different physical properties, including solubility. In general, one diastereomeric salt crystallizes and the other one stays in solution. A further recrystallization from the same solvent or solvent mixture affords the diastereomeric salt with the desired diastereoisomeric purity. The salt is then split giving the free desired enantiomer and the resolving agent is optionally recovered. The unwanted enantiomer is tipically released, racemised and recycled through the process.
In a typical resolution process for (S)-Pregabalin, the racemic mixture is combined with the chiral resolving agent in a suitable solvent or solvent mixture, and the suspension is heated until complete dissolution is achieved. Then the system is cooled in order to crystallize one diastereoisomeric salt.

The Patent EP 830 338 discloses a process for the resolution of racemic Pregabalin, which involves selective crystallization of a diastereoisomeric salt of racemic Pregabalin with the chiral resolving agent (S)-(+)-mandelic acid. The disclosed method requires the synthesis of 2-carboxyethyl-3-cyano-5-methylhexanoic acid ethyl ester, an intermediate of Pregabalin, by using a toxic cyanide source (hydrogen cyanide, sodium cyanide, potassium cyanide, or magnesium cyanide).

The method described in the Patent Application EP 1 472 210, includes a process for producing substituted γ-amino acids, such as Pregabalin, where a substituted acrylic acid ester, (2E)-5-methylhex-2-ene carboxylic acid ethyl ester, is condensed with nitromethane to yield 5-methyl-3-nitromethylhexane carboxylic acid ethyl ester, potentially hazardous. Hydrogenation of 5-methyl-3-nitromethylhexane carboxylic acid ethyl ester to 4-isobutylpyrrolidin-2-one, with a toxic and flammable Nickel catalyst, and following hydrolysis, give Pregabalin. The use of the unstable nitromethane, the hydrogenation with a toxic and flammable Nickel catalyst, the need of an optical resolution raise serious problems in adapting this process for production scale.

(S)-Pregabalin has been prepared also by asymmetric synthesis.

The method described in U.S. Patent 5,563,175 includes the use of the pyrophoric strong base n-butyllithium at low temperatures (< -35 °C) under carefully controlled conditions. This synthetic route requires the use of (4R,5S)-4-methyl-5-phenyl-2-oxazolidinone as a chiral auxiliary to introduce the desired stereochemical configuration in the final product. Also in U.S. Patent Nos. 6,359,169, 6,028,214,5, 847,151, 5,710,304,5, 684,189, 5,608,090, and 5,599, 973, (S)-Pregabalin has been synthesized directly using the chiral auxiliary (4R,5S)-4-methyl-5-phenyl-2-oxazolidinone. Although these methods provide (S)-Pregabalin in high enantiomeric purity, they are less desirable for large-scale synthesis because they employ costly reagents (e.g. the chiral auxiliary) that are difficult to handle, as well as special cryogenic equipment to reach the required operating temperatures, which can be as low as -78 °C.

(S)-Pregabalin may be prepared according to the process disclosed in EP 1 250 311, by an asymmetric hydrogenation of a cyano- substituted olefin to produce a chiral cyano precursor of (S)-Pregabalin, which is further reduced to obtain (S)-Pregabalin. The application discloses the use of various C₂-symmetric bisphosphine ligands, including (R,R)-Me-DUPHOS, which results in substantial enrichment of (S)-Pregabalin over (R)-Pregabalin. Further improvements would be desirable for many reasons. Chiral ligands such as C₂-symmetric bisphosphines, including the proprietary ligand (R,R)-Me-DUPHOS, often require several synthetic steps to be prepared with significant impact on the final cost of the product. Furthermore, the disclosed method requires the use of the carcinogenic acrylonitrile, the use of highly toxic carbon monoxide under high pressure, the reduction of the nitrile with a toxic and flammable Nickel catalyst, raising serious problems in adapting this process for production scale.

Thus, although these general strategies provide the target compound in high enantiomeric purity, they are not practical for large-scale synthesis because they employ reagents which are either expensive or difficult to handle or both, as well as specialized equipment to reach the required operating conditions. These drawbacks make them ill-suited to be employed on common multipurpose industrial plants.

Because (S)-Pregabalin is being developed as a commercial pharmaceutical product, the need exists for an efficient, cost effective, and safe method for its large scale synthesis that overcomes the limitations of the above procedures.

An object of the present invention is to provide novel compounds, which are especially useful as intermediates for the preparation of Pregabalin. The invention also relates to process for preparing these intermediates and a novel, efficient, economical and commercially useful stereoselective process for making Pregabalin using cheap and safe starting materials.

### Summary of the invention

The method of the present invention involves a process for preparing a compound of Formula (VI), a precursor of Pregabalin, 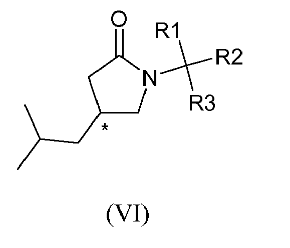 wherein:
R₁, R₂ and R₃ are each independently hydrogen, C₁-C₆ alkyl, C₃-C₆ cycloalkyl, aryl, heteroaryl, optionally substituted;
   which comprises:
   hydrogenating a compound of Formula (V), 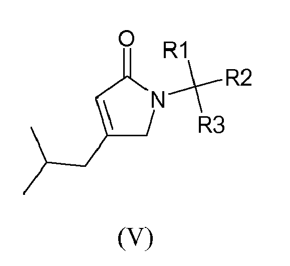 wherein R₁, R₂ and R₃ are as above defined, in the presence of a catalyst.

In another embodiment, the invention provides a process for preparing a compound of Formula (V), by cyclization of a compound of Formula (IV) 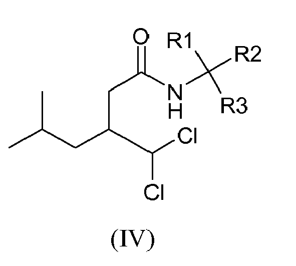 wherein R1, R2 and R3 are as defined above.

In another embodiment, the invention provides a process for preparing a compound of Formula (IV), by reacting 2,2-dichloro-3-isobutylcyclobutanone of Formula (II) 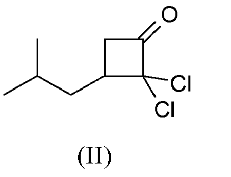 with a primary amine of Formula (III), 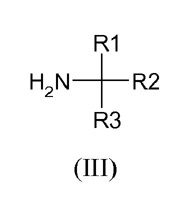 wherein R1, R2 and R3 are as defined above.

In another embodiment, the invention provides a compound of Formula (VI), as defined above, or a hydrate, a solvate thereof, with the proviso that said compound is not (4S)-isobutyl-1-((1S)-phenylethyl)-pyrrolidin-2-one, nor (4R)-isobutyl-1-((1S)-phenylethyl)-pyrrolidin-2-one.

In another embodiment, the invention provides a compound of Formula (V), as defined above, or a hydrate, a solvate thereof.

In another embodiment, the invention provides a compound of Formula (IV), as defined above, or a hydrate, a solvate thereof.

In another embodiment, the invention provides a compound of Formula (II), as defined above, or a hydrate, a solvate thereof.

In accordance with the present invention, it has been discovered that the employment of novel compounds of Formula (II), (IV), (V) and (VI), described below as intermediates for the preparation of pharmaceutically effective Pregabalin, substantially cures or remedies all of the deficiencies associated with the previously known processes.

In another embodiment, the invention provides the use of compounds of Formula (II), (IV), (V) and (VI), as intermediates in the preparation of Pregabalin.

In another embodiment, the invention provides a process for preparing Pregabalin, or a pharmaceutically acceptable salt, hydrate, solvate, or polymorph thereof, the process comprising hydrogenating a compound of Formula (V), as defined above, in the presence of a catalyst, to give a compound of Formula (VI), as defined above.

The present invention offers several advantages over previous methods of making Pregabalin. For example, the present process does not require the use of hazardous nitro or cyano compounds, the carcinogenic acrylonitrile, the toxic carbon monoxide, costly chiral auxiliaries; or low temperatures, as required in previous methods. Furthermore, processing to remove the undesired (R)-enantiomer and subsequent disposal of this waste is minimized. The present method is conducted under mild conditions and it makes use of generally inexpensive and readily available reagents.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "C₁-C₆ alkyl" refers to a straight or branched hydrocarbon having from 1 to 6 carbon atoms, preferably 1 to 4 carbon atoms, more preferably up to 2 carbon atoms. Examples of alkyl groups include, without limitation, methyl, ethyl, *n*-propyl, isopropyl, *n-*butyl, *sec*-butyl, isobutyl, *tert*-butyl, *n*-pentyl, *n*-hexyl groups and the like.

The term "C₃-C₁₀ cycloalkyl" refers to a saturated or unsaturated carbocyclic group of from 3 to 10 carbon atoms having a single ring (*e*.*g*., cyclopropyl, cyclobutyl, cyclopentyl, cyclohexyl or cyclohexenyl). Examples of C₃-C₁₀ cycloalkyl include, without limitation, cyclopentyl, cyclohexyl, and the like.

The term "aryl" refers to an aromatic carbocyclic group having a single ring (e.g., phenyl), multiple rings (e.g., biphenyl), or multiple condensed rings in which at least one is aromatic (*e*.*g*., 1,2,3,4-tetrahydronaphthyl, 1-naphthyl, 2-naphthyl, anthryl, or phenanthryl). The aryl group may be optionally substituted. A preferable aryl group of the present invention is phenyl, 1-naphtyl, or 2-naphtyl, more preferably phenyl.

The term "heteroaryl" refers to a monocyclic heteroaromatic, or a bicyclic or a tricyclic fused-ring heteroaromatic group. Examples of heteroaromatic groups include, without limitation, pyridyl, pyrrolyl, pyrimidinyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, 1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazolyl, benzoxazolyl, quinolizinyl, quinazolinyl, pthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl or benzoquinolyl. The heteroaryl group may be optionally substituted.

The term "substituted" refers to groups in which one or more hydrogen atoms have been replaced with one or more substituents selected from the group consisting of C₁-C₆ alkyl, C₃-C₁₀ cycloalkyl, aryl, heteroaryl, halogen, amino, aminosulfonyl, ammonium, aminocarbonyl, sulfinyl, sulfanyl, sulfonyl, hydroxy, alkoxy, carboxy, alkoxycarbonyl, carbamate, trihalomethyl, cyano, mercapto, nitro, and the like.

The term "lower alcohol" refers to straight, branched or cyclo- alkyl residues containing 1 to 6 carbon atoms with one hydroxy group, such as methanol, ethanol, *n-*propanol, *i*-propanol, *n*-butanol, *sec*-butanol, isobutanol, *tert*-butanol, 1-pentanol, 2-pentanol, *iso*-amyl alcohol, *tert*-amyl alcohol, cyclopentanol, 1-hexanol, 2-hexanol, 3-hexanol or cyclohexanol and the like.

The compounds prepared by the process of the present invention may have one or more stereogenic centers and may exist and may be used or isolated in optically active (enantiomeric or diastereomeric) and racemic forms. It is to be understood that the processes of the present invention can give rise to any racemic or optically-active forms, or mixtures thereof. It is to be further understood that the products of the invention process can be isolated as racemic, or optically active forms, or mixtures thereof. Purification and characterization procedures for such products are known to those of ordinary skill in the art, and include recrystallization techniques, as well as chiral chromatographic separation procedures as well as other methods.

Certain formulae may also include one "*" (asterisk) to indicate stereogenic (chiral) center, although the absence of asterisks does not indicate that the compound lacks one stereocenter. Such formulae may refer to the racemate or to individual enantiomers or diastereomers, which may or may not be substantially pure.

The term "Pregabalin" refers to racemic-Pregabalin, or (S)-Pregabalin.

The term "(S)-Pregabalin" refers to enantiomerically pure (S)-Pregabalin, or enantiomerically enriched (S)-Pregabalin.

The term "racemic" refers to a sample of a chiral compound which contains both the (+) and (-) isomers in equal amounts.

The term "enantiomerically enriched" refers to a sample of two enantiomers having an enantiomeric ratio *(i.e.* the ratio between the two enantiomers) of, for example >50 : <50, preferably ≥60 : ≤40, more preferably >80 : ≤ 20.

The term "enantiomerically pure" refers to a sample containing greater than about 95% of the desidered enantiomer, preferably greater than about 98% of the desidered enantiomer, and more preferably greater than about 99.5% of the desidered enantiomer. For example, the enantiomerically pure (S) -Pregabalin is substantially free of (R)-Pregabalin. The term "substantially free" means that a diastereoisomer or enantiomer contains less than about 5%, preferably less than about 2%, and more preferably less than about 0.5% of the other enantiomer.

The term "diastereomerically enriched" refers to a sample of two diastereoisomers having an diastereomeric ratio (i.e.the ratio between the two diastereoisomers) of, for example >50 : <50, preferably >60 : ≤40, more preferably >80 : ≤ 20.

The term "diastereomerically pure" refers to a sample containing greater than about 95% of the desidered diastereoisomer, preferably greater than about 98% of the desidered diastereoisomer, and more preferably greater than about 99.5% of the desidered diastereoisomer.

The term "enantioselectivity" refers to a given reaction (e.g., hydrogenation) that yields enantiomerically enriched products.

The term "diastereoselectivity" refers to a given reaction (e.g., hydrogenation) that yields diastereomerically enriched products.

The term "about" encompasses the range of experimental error that may typically occur in a measurement.

The term "pharmaceutically acceptable salt(s)", as used herein, unless otherwise indicated, includes salts of acidic or basic groups which may be present in the compounds of the present invention. The compounds prepared according to the present invention that are basic in nature are capable of forming a wide variety of salts with various inorganic and organic acids. The acids that may be used to prepare pharmaceutically acceptable acid addition salts of such basic compounds of are those that form non-toxic acid addition salts i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts. The compounds prepared according to the present invention that include a basic moiety, such as an amino group, may form pharmaceutically acceptable salts with various amino acids, in addition to the acids mentioned above.
Those compounds prepared according to the present invention that are acidic in nature are capable of forming base salts with various pharmacologically acceptable cations.
Examples of such salts include the alkali metal or alkaline earth metal salts and, particularly, the calcium, magnesium, sodium and potassium salts or ammonium, for example *tert*-butylammonium and substituted ammonium or amino acids salts, for example lysine, of the compounds of the present invention.

The term "hydrate" refers to a solvate comprising a disclosed or claimed compound and a stoichiometric or non-stoichiometric amount of water.

The term "solvate" refers to a molecular complex comprising a disclosed or claimed compound and a stoichiometric or non-stoichiometric amount of one or more solvent molecules (e.g., ethanol).

Generally, the chemical transformations described throughout the specification may be carried out using substantially stoichiometric amounts of reactants, though certain reactions may benefit from using an excess of one or more of the reactants. Additionally, many of the reactions disclosed throughout the specification, may be carried out at room temperature, but particular reactions may require the use of higher or lower temperatures, depending on reaction kinetics, yields, and the like. Furthermore, many of the chemical transformations may employ one or more compatible solvents, which may influence the reaction rate and yield. Depending on the nature of the reactants, the one or more solvents may be polar protic solvents, polar aprotic solvents, non-polar solvents, or some combination.
The compounds obtained by the chemical transformations of the present invention, can be used for the following steps without further purification or can be effectively separated and purified by employing a conventional method well known to those skilled in the art, such as recrystallization, column chromatography, or by transforming then into a salt or by washing with an organic solvent or with an aqueous solution, eventually adjusting pH.

In accordance with Scheme 1, the present invention provides novel compounds of Formula (II), (IV), (V) and (VI), as described above, wherein R₁, R₂ and R₃ are each independently hydrogen, C₁-C₆ alkyl, C₃-C₆ cycloalkyl, aryl, heteroaryl, optionally substituted. Preferably, the alkyl group is methyl, ethyl or propyl; more preferably methyl. Preferably, the aryl group is phenyl, 1-naphtyl, or 2-naphtyl, optionally substituted, more preferably phenyl.

Compounds of Formula (II), (IV), (V) and (VI) are intermediates for the preparation of pharmaceutically effective Pregabalin.

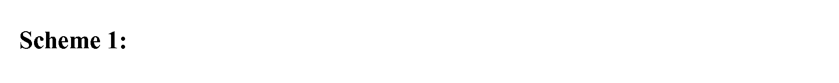

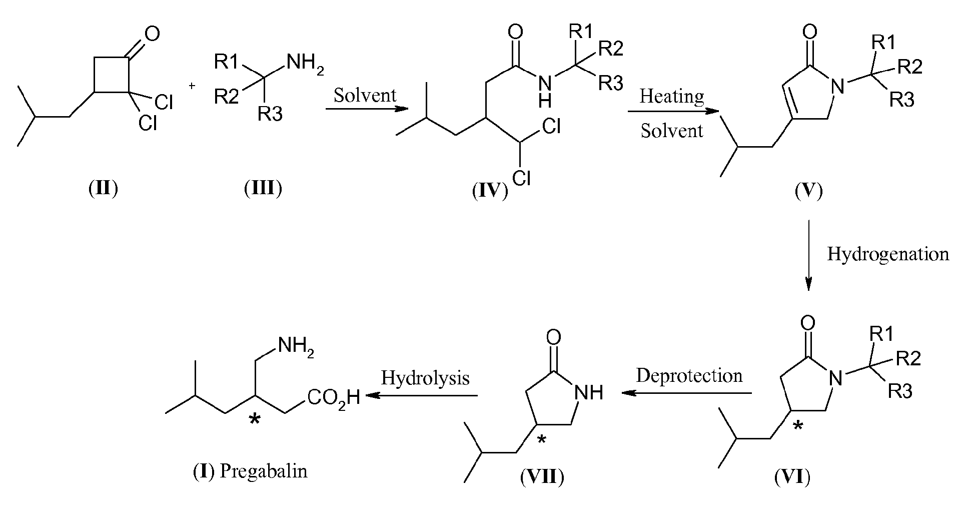

The process comprises hydrogenation of a compound of Formula (V) to produce a compound of Formula (VI), precursor of Pregabalin (Scheme 1).

In one embodiment, the invention provides the hydrogenation of a compound of Formula (V), in the presence of a catalyst. The hydrogenation can be carried out by reaction with hydrogen gas, applied at pressures ranging from about 0.1 to 100 bar above the atmospheric pressure, preferably from about 0.1 to 10 bar; or employing salts capable of generating hydrogen or hydride anions in the presence of a suitable metal catalyst, such as ammonium formate; sodium formate or potassium formate; trialkylammonium formats; formic acid; or silicon compounds containing the Si-H moiety, such as trialkylsilanes, for example triethylsilane, or hydrosiloxanes, such as poly(methylhydrosiloxane).

Any suitable solvent known to those skilled in the art can be used for hydrogenation of a compound of Formula (V). Suitable solvents are a polar protic solvent, such as water; a lower alcohol, such as methanol, ethanol, *i*-propanol, 1- or 2- or *tert*-butanol; an ester, such as ethyl acetate or isopropyl acetate; an ether, such as tetrahydrofuran, 2-methyltetrahydrofuran or 1,4-dioxane; an aliphatic or aromatic hydrocarbon, such as hexane or heptane, toluene or xylenes; a polar aprotic solvent, such as dimethylformamide, dimethylacetamide or N-methylpyrrolidone; or mixtures thereof.

The hydrogenation is done at a suitable temperature. A suitable temperature is a temperature in order for the reaction to be complete. The temperature may ranges from about 0 °C to the solvent reflux, preferably from about 10 to 50 °C, more preferably at about 25 °C.

Any suitable hydrogenation catalyst known to those skilled in the art can be used for hydrogenation. Suitable hydrogenation catalyst are heterogeneous catalysts, achiral or chiral homogeneous catalysts.

Suitable heterogeneous hydrogenation catalysts are transition metals, such as Nickel, Copper, Palladium, Platinum, Rhodium, Ruthenium, Rhenium, Iridium or Gold, or mixtures thereof, in a metallic or metal oxide or salt form; in a supported or unsupported form, such as Palladium or Platinum black, Palladium or Platinum supported on charcoal or on alumina, Palladium(II) hydroxide on carbon (Pearlman's catalyst), Palladium on barium carbonate, Palladium on barium sulfate, Palladium on calcium carbonate, Platinum(IV) oxide (Adam's catalyst), Raney Nickel or Nickel on charcoal, or Ruthenium or Rhodium black or Ruthenium or Rhodium on alumina; or in the form of metal nanoparticles; or a metallic colloid. Suitable supports are any meso- and microporous high specific surface solids such as alumina or silica or titania or zeolites or graphite or bohemite, or synthetic polymers. Preferred transition metals are commercial, economical and not hazardous Palladium or Platinum, more preferably Platinum; and a preferred support is charcoal.

Suitable achiral homogeneous catalysts are also transition metal complexes, such as (Ph₃P)₃RhCl (Wilkinson's catalyst); [(Ph₃P)₂Rh(COD)]PF₆ (Schrock-Osborn catalyst); [(PCy₃)(Py)Ir(COD)]PF₆ (Crabtree's catalyst); preferably Wilkinson's catalyst; or catalysts prepared from transition metal salts, such as [Rh(COD)_{2]}CF₃SO₃, [Rh(nbd)₂]BF₄, [RhCl(COD)]₂, [RuCl₂(η⁶-p-cymene)]₂, [(COD)Ru(η³-methallyl)₂], [IrCl(Cyclooctene)₂]₂, [Ir(COD)₂]BF₄, and achiral monodentate ligands, such as phosphines, for example triarylphosphines or triheteroarylphosphines, or achiral bidentate ligands, for example 1,2-bis(diphenylphosphino)ethane and the like.

"COD" refers to 1,4-cyclooctadiene and "nbd" refers to norbornadiene.

Suitable chiral homogeneous hydrogenation catalysts are chiral organometallic complexes of transition metals, such as Rhodium, Ruthenium or Iridium.
Said chiral complexes are formed from transition metal salts, such as [Rh(COD)_{2]}CF₃SO₃, [Rh(nbd)₂]BF₄, [RhCl(COD)]₂, [RuCb(η⁶-p-cymene)]₂, [(COD)Ru((3-methallyl)₂], [IrCl(Cyclooctene)₂]₂, [Ir(COD)₂]BF₄, in the presence of an optically active ligand, such as atropisomeric biaryl bisphosphine ligands, for example BINAP and related structures; chiral C₂ symmetric phosphorus ligands, for example DIPAMP and its analogues; phospholane ligands, for example as DUPHOS, commercially available from Strem Chemicals, Inc. (7 Mulliken Way, Newburyport, MA 01950-4098) and Chirotech Technology Limited (Cambridge Science Park, Cambridge, Great Britain) (see U.S. Patent Nos. 5,532,395 and 5,171,892, DuPont); chiral ferrocene-based bisphosphane based ligands, for example WALPHOS (see U.S. Patent n° 6,777,567, Solvias); phosphinooxazolines (PHOX ligands); phosphinitooxazolines or phosphinitoimidazolines (see U.S. Patent application 20070010493, Solvias); or chiral phosphoramidite family Monophos (see WO 200204466, DSM) of either enantiomeric form, in an optically pure form or as an enantiomerically enriched mixture.

The catalytic system, i.e. the metal salt and ligand, may be formed *in situ,* or prepared in a separate step, optionally isolated and used.

Depending on the meaning of R1, R2 and R3 and on the catalyst or catalytic system used, the hydrogenation may generate an enantiomerically or diastereomerically enriched product.

According to the invention, when a compound of Formula (V), wherein R₁, R₂ and R₃ as defined above are different and with a defined (R) or (S) stereochemistry is hydrogenated, a mixture of two diastereoisomers of Formula (VI) are obtained.
Preferably a compound of Formula (VI) is in the form of enriched or pure diastereoisomers having (S) configuration at carbon 4- of the pyrrolidinone ring as indicated below.

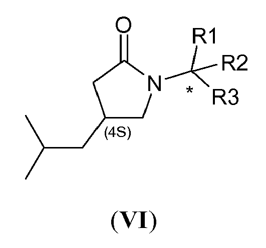

The diastereomeric purity of compound of Fomula (VI), wherein R₁, R₂ and R₃, as defined above, are different, can be further increased, if necessary, by recrystallization from a suitable solvent o solvent mixture or by forming a salt with a mineral or organic acid and crystallizing said salt from water or an organic solvent or mixtures thereof.
A mineral acid is for example hydrochloric, hydrobromic or sulfuric acid; an organic acid is for example acetic, formic, toluenesulfonic or camphorsulfonic acid; or a dicarboxylic acid, such as oxalic or maleic acid.
An organic solvent is for example a lower alcohol, such as methanol, ethanol or isopropanol; a ketone, such as acetone or methyl ethyl ketone; or an ether, such as tetrahydrofuran or 1,4-dioxane.

When a compound of Formula (V), wherein at least two groups of R₁, R₂ and R₃, as defined above, are equal, is hydrogenated, a mixture of (S) and (R) enantiomers, or single (S) or (R) enantiomers of Formula (VI) are obtained. A mixture of enantiomers of Formula (VI) can contain the two enantiomers in any ratio to each other.

The enantiomeric purity is generally expressed as "enantiomeric excess" and defined, for example, for the (S) enantiomer as (S-R)/(R+S)x100 wherein S and R are respectively the amounts of the (S) and (R) enantiomers.

Compounds of Formula (V) and compounds of Formula (VI) are also object of the present invention, with the proviso that compound of Formula (VI) is not (4S)-isobutyl-1-((1S)-phenylethyl)-pyrrolidin-2-one, nor (4R)-isobutyl-1-((1S)-phenylethyl)-pyrrolidin-2-one.

Preferred examples of the compounds of Formula (V) and Formula (VI), that are a further object of the invention, are:
1-benzyl-4-isobutyl-1,5-dihydropyrrol-2-one;
4-isobutyl-1-((1R)-phenylethyl)-1,5-dihydropyrrol-2-one;
4-isobutyl-1-((1S)-phenylethyl)-1,5-dihydropyrrol-2-one;
(4S)-isobutyl-1-benzylpyrrolidin-2-one;

A compound of Formula (V) may be carried out by cyclization of a compound of Formula (IV). The cyclization is obtained by heating a compound of Formula (IV) in a suitable solvent, optionally in the presence of a halide salt (Scheme 1). A suitable solvent is a lower alcohol, such as methanol, ethanol, *n*-propanol, *i*-propanol, *n*-butanol, *sec-*butanol, isobutanol, *tert*-butanol, 1-pentanol, 2-pentanol, isoamyl alcohol, *tert*-amyl alcohol, cyclopentanol, 1-hexanol, 2-hexanol, 3-hexanol or cyclohexanol, preferably an alcohol with a boiling point above 100 °C at atmospheric pressure. The solvent is employed in a volume to weight ratio to compound (IV) ranging from about 1 to 10, preferably from about 4 to 6.
The reaction is performed at a temperature ranging from room temperature to the reflux temperature of the mixture, preferably from about 50 °C to the reflux temperature of the mixture, more preferably at reflux.
An optional halide salt is lithium bromide or iodide; sodium, potassium, magnesium or calcium iodide , preferably potassium iodide. The molar ratio between a compound of Formula (IV) and a halide salt is in the range of about 1 to 50 mol %, preferably of about 10 to 20 mol %.

Compounds of Formula (IV) are novel compounds. Preferred examples of the compounds of Formula (IV) that are a further object of the invention are listed below:
*N*-benzyl-3-dichloromethyl-5-methylhexanamide;
*N*-((1R)-phenylethyl)-3-dichloromethyl-5-methylhexanamide;
*N*-((1S)-phenylethyl)-3-dichloromethyl-5-methylhexanamide.

A compound of Formula (IV), may be obtained by reacting a compound of Formula (II) with a primary amine (III), wherein R₁, R₂ and R₃ are as defined above (Scheme 1).

The primary amine (III) used in the process of the present invention may have one or more stereogenic centers and may exist and may be used in optically active or racemic forms. It is to be understood that the process of the present invention can employ any racemic, optically active, or stereoisomeric form, or mixtures thereof, of a primary amine (III).

Examples of a primary amine (III), include, without limitation, methylamine, ethylamine, n-propylamine, isopropylamine, n-butylamine, isobutylamine, *tert-*butylamine, triphenylmethylamine, 1-methyl-1-phenylethylamine, benzylamine, (R)-, (S)- or (*rac*)-1-phenylethylamine, (R)-, (S)- or (*rac*)-1-napht-1-ylethylamine, (R)- or (S)- or (*rac*)- 2-napht-1-ylethylamine, (R)-, (S)- or (*rac*)-2-phenylglycinol or any isomer of 1-amino-2-indanol. Preferably the primary amine (III) is methylamine, ethylamine, *n*-propylamine, *tert*-butylamine, benzylamine or (R)- or (S)-1-phenylethylamine; more preferably benzylamine or (R)- or (S)-1-phenylethylamine.

The primary amines described above are commercially available compounds.

The reaction of the primary amine of Formula (III) and compound of Formula (II) may be carried out in a dipolar aprotic organic solvent, such as an ether, for example diethyl ether, methyl *tert*-butyl ether, tetrahydrofuran, 2-methyltetrahydrofuran or 1,4-dioxane; a nitrile, for example acetonitrile; an amide, for example N,N-dimethylacetamide or N,N-dimethyl formamide. Preferably the reaction is carried out in an ether, such as tetrahydrofuran. The molar ratio between the compound of Formula (II) and a primary amine (III) is comprised between 1 and 3 equivalents, preferably between 1.5 and 2 equivalents. The ring opening reaction of compound of Formula (II) with a primary amine of Formula (III) can be carried out at a temperature ranging between about -20 °C and +30 °C, preferably between about -10 °C and +20 °C, more preferably at about 0°C.

The starting material of the process of this invention, 2,2-dichloro-3-isobutylcyclobutanone (II), is readily available. It can be prepared by [2+2] cycloaddition of the commercially available 4-methyl-1-pentene and dichloroketene following methods well known in the art, for instance Krepski, L. et al., J. Org. Chem. 1978, 43, 2879.

Dichloroketene may be generated by any method known in the art, for instance Organic Syntheses, Coll. Vol. 8, p.82 (1993) or Organic Syntheses, Coll. Vol. 6, p.1037 (1988).

2,2-dichloro-3-isobutylcyclobutanone (II) is a novel compound and is a further object of the invention.

In another embodiment, the present invention provides use of compounds of Formula (II), (IV), (V) or (VI), as defined above, as intermediates in the preparation of Pregabalin.

In another embodiment, the present invention provides an efficient method of preparing Pregabalin, or a pharmaceutically acceptable salt, hydrate, solvate, or polymorph thereof, particularly suited for large scale preparation (Scheme 2).

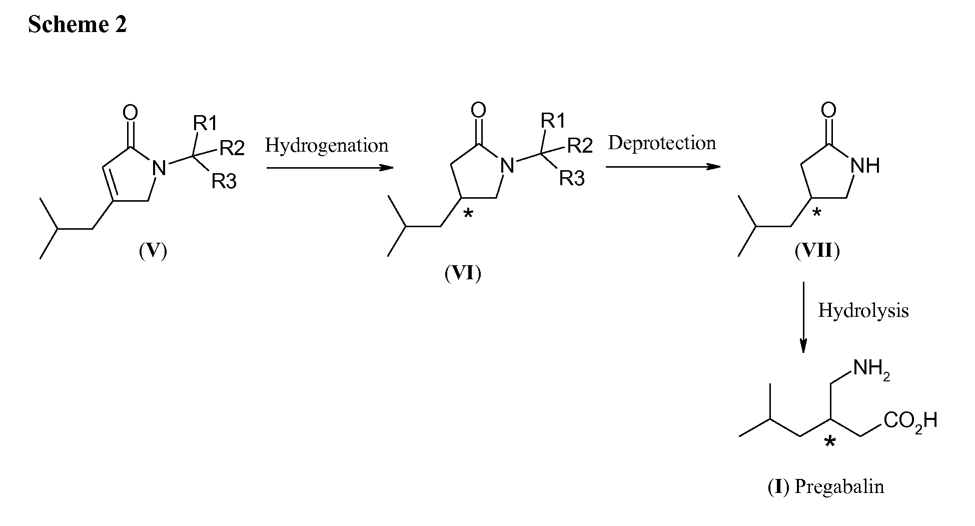

As described in Scheme 2, Pregabalin may be prepared by the above described process of hydrogenation of compound of Formula (V), in the presence of a catalyst, to give a compound of Formula (VI).

A compound of Formula (VI), in particular the single (4S)- isomer, is particularly useful in the preparation of Pregabalin.

Said compound of Formula (VI), can be converted to Pregabalin in two different ways:
a) by nitrogen deprotection of a compound of Formula (VI), to give a compound of Formula (VII) and following hydrolysis of a compound of Formula (VII) to Pregabalin (Scheme 2);
   or
b) by hydrolysis or alcoholysis of a compound of Formula (VI), to give a compound of Formula (VIII) and following nitrogen deprotection of a compound of Formula (VIII) to Pregabalin (Scheme 3). 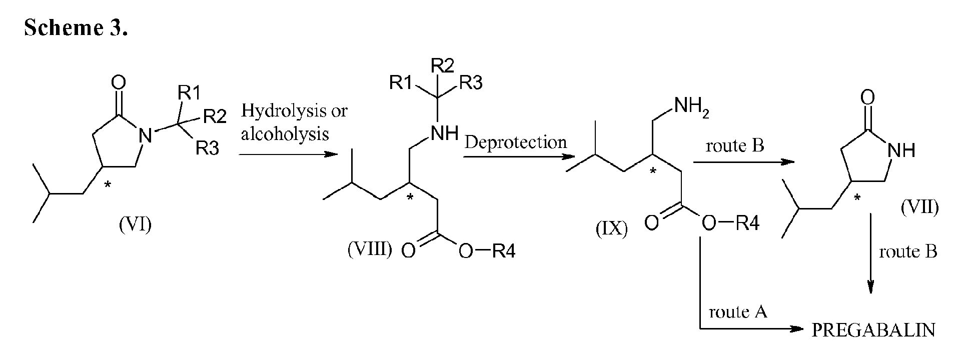 According to Scheme 2, a compound of Formula (VI) may subjected to nitrogen deprotection, thereby obtaining the lactam form of Pregabalin (VII), which can be in turn converted to Pregabalin, by means of hydrolysis.
   Nitrogen deprotection of compound of Formula (VI), when at least one of the R₁ or R₂ or R₃ groups is an aryl or heteroaryl group , can be carried out directly by hydrogenolysis, as described above for the hydrogenation of a compound of Formula (V), in a one-pot fashion, without isolation of a compound of Formula (VI), by increasing the hydrogen pressure and/or the temperature after the complete hydrogenation of the double bond of a compound of Formula (V) has been achieved; or in a stepwise fashion starting from isolated compound (VI).
   Nitrogen deprotection of a compound of Formula (VI) to give a compound of Formula (VII), can be carried out also by treatment with a Bronsted acid, optionally in a suitable solvent and with optional heating. A suitable Bronsted acid is for example an inorganic acid, such as a hydrohalic acid, for instance hydrogen chloride, hydrogen bromide or hydrogen iodide, sulfuric acid, phosphoric acid or solutions thereof in water or in a suitable solvent; organic acids, such as carboxylic acids, for example acetic acid, trichloroacetic acid or trifuoroacetic acid, sulfonic acids, such as methanesulfonic acid, trifluoromethanesulfonic acid, benzenesulfonic acid, toluenesulfonic acid, camphorsulfonic acid.
   Preferred acids are methanesulfonic acid or sulfuric acid, at a concentration comprised between about 10% in water and about 95-98%, more preferably between about 50% and 98%.
   The molar ratio of a Bronsted acid and a compound of Formula (VI) can be comprised between about 0.1 and 50 molar equivalents, preferably between about 1 and 10 molar equivalents.
   A suitable solvent is an organic solvent, such as aromatic solvent, e.g. benzene, toluene, xylenes, anisole, phenol, thiophenol or thioanisole, preferably toluene or xylenes.
   The deprotection reaction is run between room temperature and the reflux temperature of the mixture, preferably at reflux.
   Finally, hydrolysis of compound of Formula (VII), which may be carried out by methods well known in the art for the ring opening of gamma-lactams, for example as described in J. Am. Chem. Soc. 2005, 127(1), 119-125 or Tetrahedron Letters 1998, 39(1), 79-82, gave Pregabalin.
   According to Scheme 3, a compound of Formula (VI), can be converted to Pregabalin by hydrolysis or alcoholysis of a compound of Formula (VI), to give a compound of Formula (VIII) and following nitrogen deprotection of a compound of Formula (VIII) to give Pregabalin.
   Hydrolysis or alcoholysis of a compound of Formula (VI) to a compound of Formula (VIII), wherein R₄ is an alkaline or earth alkaline cation, hydrogen, or C₁-C₆ alkyl, can be carried out by treatment with a suitable base, a suitable acid or an alcohol salt.
   A suitable base is an hydroxide salt of an alkaline or alkaline earth element, such as sodium hydroxide, potassium hydroxide, magnesium hydroxide or calcium hydroxide, preferably sodium or potassium hydroxide.
   A suitable acid is a mineral acid such as hydrochloric or sulfuric acid, preferably hydrochloric acid.
   An alcohol salt is the alkaline or alkaline earth salt of a lower alcohol, such as sodium or potassium methoxide, sodium or potassium ethoxide, sodium or potassium *tert*-butoxide, preferably sodium or potassium methoxide.
   Finally, a compound of Formula (VIII) can be in turn converted to Pregabalin, by nitrogen deprotection via a compound of Formula (IX) and optional ester hydrolysis (Scheme 3, route A) or via lactam of Formula (VII) (Scheme 3, route B), according to the conditions described above.
   Racemic Pregabalin, enantiomerically enriched or enantiomerically pure (S)-Pregabalin may be obtained with the process of the present invention. It depends on, among other things, from the choice of the primary amine (III) and/or the catalyst and/or solvent used for the hydrogenation of compound of Formula (V).
   For instance, racemic Pregabalin is obtained when benzylamine is used to open 2,2-dichloro-3-isobutylcyclobutanone (II), and metal heterogeneous catalyst is used during the hydrogenation of compound of Formula (V), Schemes 1 and 2.
   (S)-Pregabalin is obtained when benzylamine is used to open 2,2-dichloro-3-isobutylcyclobutanone (II), and a transition metal chiral homogeneous catalyst is used during the hydrogenation of compound of Formula (V), Schemes 1 and 2.
   (S)-Pregabalin is also obtained when the cheap optically active (R)-1-phenylethylamine is used to open 2,2-dichloro-3-isobutylcyclobutanone (II), and a heterogeneous metal catalyst is used during the hydrogenation of compound of Formula (V), Schemes 1 and 2.
   When racemic Pregabalin is obtained, the desidered (S)-Pregabalin can be obtained by classical optical resolution of racemic Pregabalin. The racemic mixture is combined with a chiral resolving agent in a suitable solvent or solvent mixture, and the suspension is heated until complete dissolution is achieved. Then the system is cooled in order to crystallize one diastereoisomeric salt. A chiral resolving agent is for example camphorsulfonic acid, mandelic acid, tartaric acid, dibenzoyltartaric acid, di p-toluoyltartaric acid.
   The enantiomers of Pregabalin can contain the two enantiomers in any ratio to each other. Preferably, Pregabalin is in the enantiomerically pure form. More preferably, Pregabalin is (S)-Pregabalin.
   The desidered (S)-Pregabalin m ay be further enriched through classical resolution, chiral chromatography or recrystallization. For example, the (S)-Pregabalin may be reacted with an enantiomerically pure compound (e.g. acid or base) to yield a pair of diastereoisomers, each composed of a single enantiomer, which are separated via fractional recrystallization or chromatography. The desidered enantiomer is subsequently regenerated from the appropriate diastereoisomer. Additionally, the desidered enantiomer often may be further enriched by recrystallization in a suitable solvent.
   Many of the compounds described in this disclosure, are capable of forming pharmaceutically acceptable salts. These salts include, without limitation, acid addition salts (including diacids) and base salts.
   One may prepare a pharmaceutically acceptable acid addition salt (or base salt) by contacting a compound's free base (or free acid) or zwitterion with a sufficient amount of a desired acid (or base) to produce a nontoxic salt. If the salt precipitates from solution, it may be isolated by filtration; otherwise, the salt may be recovered by evaporating the solvent.
   One may also regenerate the free base (or free acid) by contacting the acid addition salt with a base (or the base salt with an acid). Though certain physical properties of the free base (or free acid) and its respective acid addition salt (or base salt) may differ (e.g., solubility, crystal structure, hygroscopicity, etc.), a compound's free base and acid addition salt (or its free acid and base salt) are otherwise the same for purposes of this disclosure.
   As mentioned above the compound of the invention has useful therapeutic properties. (S)-Pregabalin may be administered per se or, preferably, as a pharmaceutical composition.
   It is contemplated that the compounds of the present method can be found or isolated in the form of hydrates or solvates, in different polymorphic forms, i.e., different crystalline forms, which are considered to fall within the scope of the present invention.
   While the present invention has been described in terms of its specific embodiments, certain modifications and equivalents will be apparent to those skilled in the art and are included within the scope of the present invention.
   In the following, the present invention shall be illustrated by means of some examples, which are not construed to be viewed as limiting the scope of the invention.

### Examples

The following abbreviations refer respectively to the definitions below:
THF (tetrahydrofuran), TLC (thin layer chromatography), Bn (benzyl), Ph (phenyl) and Rₜ (retention time).

### Example 1

***N*-((1R)-Phenylethyl)-3-dichloromethyl-5-methylhexanamide (IVa)**

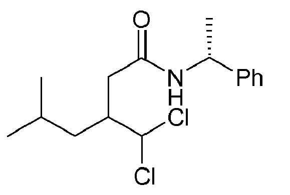

In a 300 mL jacketed glass reactor, 3-isobutyl-2,2-dichlorocyclobutanone (II) (6.8 g, 34.9 mmol) was dissolved in THF (70 mL) and cooled to -5 °C. (R)-1-phenylethylamine (7.8 g, 64.4 mmol) was dissolved in THF (70 mL) and added over two hours to the stirred solution of the ketone (II) keeping the temperature below 0 °C. The mixture was stirred 18 hours at 0 °C and quenched in 100 mL of 1N HCl. THF was evaporated under reduced pressure and toluene (100 mL) was added. The aqueous phase was discarded and the organic layer was washed with 1N HCl (50 mL), twice with brine (50 mL), dried over sodium sulphate and concentrated to an oil which solidified on standing, yielding the title compound (IVa) (10.5 g). Yield: 95%.

¹H NMR (300 MHz, CDCl₃): 50:50 mixture of diastereoisomers 0.88 and 0.92 (2 x t, J=6.0, 2 x 6H) 1.23-1.35 (m, 2 x 1H), 1.42-1.65 (m, 2 x 2H), 1.47 and 1.49 (2 x d, J=7.0, 2 x 3H), 2.20-2.35 (m, 2 x 1H), 2.45-2.55 (m, 2 x 1H), 2.62-2.73 (m, 2 x 1H), 5.11 and 5.13 (2 x q, J=7.0, 2 x 1H), 5.86 and 5.88 (2 x bs, 2 x 1H), 5.96 and 6.02 (2 x d, J=2.8, 2 x 1H), 7.25-7.34 (m, 2 x 5H)

¹³C NMR (75 MHz, CDCl₃): 50:50 mixture of diastereoisomers 13.9, 19.0, 22.3 22.8, 22.9 25.1, 25.2, 34.8, 37.3, 39.2, 43.6, 48.9, 62.4, 77.5, 126.2, 127.3, 128.6, 143.3, 170.4

### Example 2

***N*-Benzyl-3-dichloromethyl-5-methylhexanamide (IVb)**

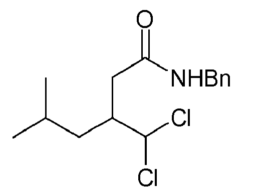

The title compound (IVb) was obtained in 95% yield using benzylamine instead of (R)-phenylethylamine and employing the same conditions described in Example 1.

¹H NMR (300 MHz, CDCl₃): 0.85-0.94 (m, 6H), 1.27-1.36 (m, 1H), 1.48-1.66 (m, 2H), 2.32 and 2.54 (2 x dd, AB system, J=15.0, 6.4, 2 x 1H), 2.70-2.76 (m, 1H), 4.46 (d, J=5.5, 2H), 5.9 (bs, 1H), 6.04 (d, J=2.4, 1H), 7.26-7.32 (m, 5H)

¹³C NMR (75 MHz, CDCl₃): 22.5, 22.9, 25.2, 37.4, 39.4, 43.6, 43.8, 76.7, 127.7, 127.9, 128.9, 157.4, 170.7

### Example 3

**4-Isobutyl-1-((1R)-phenylethyl)-1,5-dihydropyrrol-2-one (Va)**

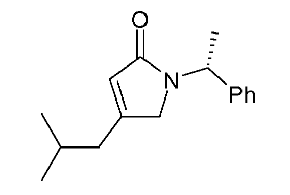

In a 100 mL round bottomed flask fitted with thermometer and reflux condenser, N-((1R)-phenylethyl)-3-dichloromethyl-5-methylhexanamide (IVa) (3.0 g, 9.5 mmol), prepared as in Example 1, was dissolved in n-butanol (20 mL) and KI (0.3 g, 1.8 mmol, 0.19 eq) was added.
The mixture was heated at reflux under nitrogen for 12 hours, then cooled to room temperature, diluted with water (20 mL) and n-butanol was removed under reduced pressure. Toluene (50 mL) was added, the aqueous layer was discarded and the organic phase was washed three times with water (20 mL each), brine (20 mL), dried over Na₂SO₄ and evaporated to an oil, which was purified by silica gel flash chromatography eluting with AcOEt-Hexane 3:7, yielding the title compound (Va) (1.96 g) as a pale yellow oil. Yield: 85%.

¹H NMR (300 MHz, CDCl₃): 0.86-0.89 (m, 6H), 1.58 (d, J=7.0, 3H), 1.78 (nonuplet, J=6.7, 1H), 2.17 (dd, J=6.7, 1.2, 2H), 3.45 (dd, J=19.0, 1.2, 1H), 3.76 (dd, J=19.0, 1.5, 1H), 5.55 (q, J= 7.0, 1H), 5.85 (m, 1H), 7.23-7.36 (m, 5H)

¹³C NMR (75 MHz, CDCl₃): 17.7, 22.5, 27.5, 39.0, 48.7, 50.5, 122.6, 126.9, 127.4, 128.6, 141.2, 158.9, 171.7

### Example 4

**1-Benzyl-4-isobutyl-1,5-dihydropyrrol-2-one** (Vb)

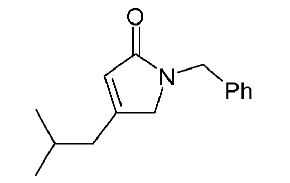

The title compound (Vb) was obtained in 80% yield as a colourless oil by cyclisation ofN-benzyl-3-dichloromethyl-5-methylhexanamide (IVb), prepared as in Example 2, in n-butanol employing the same conditions described in Example 3.

¹H NMR (300 MHz, CDCl₃) 0.89 (d, J=6.7, 6H), 1.78 (nonuplet, J=6.9, 1H), 2.18 (d, J=7.3, 2H), 3.70 (s, 2H), 4.59 (s, 2H), 5.87 (s, 1H), 7.22-7.29 (m, 5H)

¹³C NMR (75 MHz, CDCl₃) 22.5, 27.6, 39.0, 45.9, 54.2, 122.7, 128.0, 128.2, 128.8, 137.6, 159.0, 172.1

### Example 5

**1-Benzyl-4-isobutylpyrrolidin-2-one** (VIb)

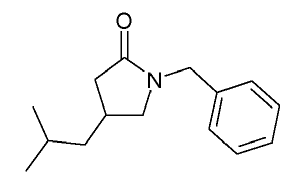

In a 3-necks round bottomed flask fitted with thermometer, a hydrogen balloon and connected to a vacuum line, 1-benzyl-4-isobutyl-1,5-dihydropyrrol-2-one (Vb) (1.00 g, 4.4 mmol), prepared as in Example 4, was dissolved in methanol (15 mL). Palladium on charcoal (10%, 50% water wet, 465 mg, 5 mol%) was added and three vacuum-hydrogen cycles were performed.
After 4 hours at 25 °C, three vacuum-nitrogen cycles were performed and the mixture was filtered on a pad of Celite^{®}. The filter was rinsed with methanol (15 mL) and the solvent was removed under reduced pressure, yielding the title compound (VIb) (0.91 g) as a colourless oil. Yield: 90%.

¹H NMR (300 MHz, CDCl₃): 0.84 (t, J=4.3, 6H), 1.22-1.27 (m, 2H), 1.50 (nonuplet, J=6.9, 1H), 2.09 and 2.56 (2 x dd, J=16.5, 8.3, 2 x 1H), 2.39 (sept., J=7.7, 1H), 2.84 and 3.30 (2 x dd, J=8.0, 7.7, 2 x 1H), 4.38 and 4.45 (AB system, J=14.7, 2 x 1H), 7.22-7.31 (m,5H)

¹³C NMR (75 MHz, CDCl₃): 21.8, 26.0, 29.7, 38.0, 44.1, 46.5, 53.2, 127.1, 128.5, 130.7, 136.0, 174.5

### Example 6

**(*Rac*)-4-Isobutylpyrrolidin-2-one (VII)**

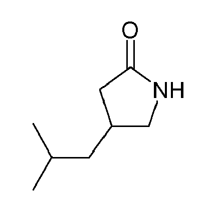

A 300-mL glass autoclave was loaded with 1-benzyl-4-isobutyl-1,5-dihydropyrrol-2-one (Vb) (5.00 g, 21.6 mmol), prepared as in Example 4, and methanol (50 mL). Palladium on charcoal (10%, 50% water wet, 1.0 g, 2.2 mol%) was added and three vacuum-nitrogen cycles were performed.
The autoclave was pressurized with hydrogen (5.0 bar) and vented three times, then pressurized to 5.0 bar and the mixture was magnetically stirred for 2 hours at 25 °C.
After venting and purging the autoclave with nitrogen, the reaction mixture was filtered on a pad of Celite^{®}. The filter was rinsed with methanol (15 mL) and the solvent was removed under reduced pressure, yielding the title compound (VII) (2.6 g) as a low-melting colourless solid.
Yield: 85%.

¹H NMR (300 MHz, CDCl₃): 0.88 and 0.89 (dd, J=3.4, 2 x 3H), 1.31 (t, J=6.1, 2H), 1.55 (sept, J=6.7, 1H), 1.96 (dd, J=16.5, 8.6, 1H), 2.35-2.47 (m, 1H), 2.52 (sept, J=7.7, 1H), 2.97 (dd, J=9.2, 7.0, 1H), 3.45 (dd, J=8.8, 7.7, 1H), 6.16 (bs, 1H)

¹³C NMR (75 MHz, CDCl₃): 22.6, 22.8, 26.2, 33.1, 37.1, 43.9, 48.4, 178.7

### Example 7

**4-Isobutyl-1-((1R)-phenylethyl)-pyrrolidin-2-one (VIa)**

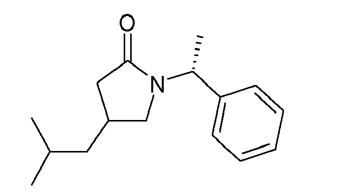

A 3-necks round bottomed flask fitted with thermometer, rubber septum and connected to a vacuum line was charged with Platinum on charcoal (10%, 160 mg, 2% mol) and three vacuum-nitrogen cycles were performed. 4-Isobutyl-1-((1R)-phenylethyl)-1,5-dihydropyrrol-2-one (Va) (1.0 g, 4.1 mmol), prepared as in Example 3, dissolved in methanol (10 mL) was added via syringe and the rubber septum was replaced by a hydrogen ballon. Three vacuum-hydrogen cycles were performed and the mixture was stirred at 25 °C for 3 hours. Three vacuum-nitrogen cycles were then performed and the mixture was filtered on a pad of Celite^{®}. The filter was rinsed with methanol (15 mL) and the solvent was removed under reduced pressure, yielding the title compound (950 mg) as a pale yellow oil with a diastereoisomeric ratio of 78:22, the major diastereoisomer having the (4S)- configuration.
Yield: 94%.

Diasteremeric ratio was determined either by ¹H NMR or by HPLC analysis (Symmetry C18 5 µm, 4.6x250 mm. Flow 0.4 mL/min. Phase A: 0.1% TFA in water. Phase B: isopropanol. Isocratic elution: 55% A, 45% B. Detector 210 nm.):
Rₜ diastereoisomer 1 = 27.8 min;
Rₜ diastereoisomer 2 = 30.1 min.

### Example 7a

Using the procedure reported in Example 7 and employing ethanol as solvent and Palladium on Charcoal (10%, 50% water wet, 5% mol), complete conversion was observed in 4 hours, with the following diastereomeric ratio: 37: 63, the major diastereoisomer having the (4R)- configuration.

### Example 7b

Using the procedure reported in Example 7 and employing THF as solvent and Palladium on Charcoal (10%, 50% water wet, 5% mol), complete conversion was observed in 8 hours, with the following diastereomeric ratio: 55: 45, the major diastereoisomer having the (4 S)- configuration.

### Example 7c

Using the procedure reported in Example 7 and employing methanol as solvent and Rhodium on charcoal (5%, 5% mol) in a 300 mL glass autoclave at 1 bar hydrogen pressure, complete conversion was observed in 2 hours, with the following diastereomeric ratio: 66:33, the major diastereoisomer having the (4S)- configuration.

### Example 7d

Using the procedure reported in Example 7 and employing THF as solvent and Wilkinson's catalyst (5%) in a 300 mL glass autoclave at 3 bar hydrogen pressure, complete conversion was observed in 4 hours, with the following diastereomeric ratio: 61:39, the major diastereoisomer having the (4S)- configuration.

### Example 7e

A 300 mL glass autoclave was charged with 4-isobutyl-1-((1R)-phenylethyl)-1,5-dihydropyrrol-2-one (Va) (250 mg, 1.03 mmol), prepared as in Example 3, and methanol (10 mL). The resultant solution was sparged with nitrogen for 15 min, then added of a solution of (R,R)-(-)-1,2-bis[(o-methoxyphenyl)(phenyl)phosphino]ethane(1,5-cyclooctadiene)Rhodium(I) tetrafluoborate (20 mg, 0.026 mmol) in 1 mL of degassed methanol.
After the autoclave was pressurized three times with hydrogen and vented, hydrogen was charged at 9 bar.
Complete conversion was observed in 48 hours, with the following diastereomeric ratio: 75:25, the major diastereoisomer having the (4S)- configuration.

### Example 8

**(4S)-(+)-Isobutylpyrrolidin-2-one (VII)**

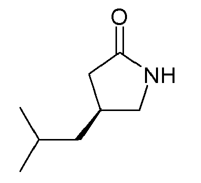

A 50-mL round bottomed flask fitted with a reflux condenser was loaded with 4-isobutyl-1-((1R)-phenylethyl)-pyrrolidin-2-one (VIa) (78:22 diastereoisomeric mixture) (2.0 g, 8.1 mmol), prepared as in Example 7, methanesulfonic acid (4.0 g, 41.6 mmol) and toluene (5 mL). The mixture was vigorously stirred and heated at reflux for 14 hours. The progress of the reaction was monitored by TLC eluting with ethyl acetate and staining with KMnO₄. After complete deprotection the mixture was cooled and carefully quenched into aqueous bicarbonate and extracted into ethyl acetate after pH adjustment to 8-9 . The organic layer was washed twice with water, brine and concentrated to residue under reduced pressure. The resulting oil was purified by silica gel chromatography eluting with ethyl acetate, yielding the title compound (VII) (1.10 g) as a colourless oil.
Yield: 96%.
¹H NMR and ¹³C NMR spectra were identical to those of the racemic material reported above (Example 6)
[α]_{D}=+1.6° (c=2.77, MeOH)
For comparison an analytical sample of (4S)-(+)-Isobutylpyrrolidin-2-one was prepared from enantiomerically pure (S)-(+)-Pregabalin.
[α]_{D}=+2.4° (c=1.08, MeOH)

### Example 9

**(S)-(+)-3-aminomethyl-5-methyl-1-hexanoic** acid (Pregabalin) (I)

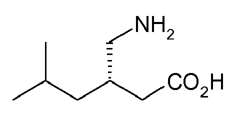

(4S)-(+)-Isobutylpyrrolidin-2-one (VII), prepared as in Example 8 (1.0 g, 7.1 mmol) was dissolved in 10 mL of 6N HCl and heated to reflux for 12 hours, then cooled to room temperature. The pH was adjusted to 7 by addition of 1N NaOH, the solid was filtered, washed with H₂O and dried, yielding the title product (1.0 g).
HPLC analysis after derivatization with Marfey's reagent and isocratic elution with 0.05 M triethylamine (adjusted to pH 3 with phosphoric acid)/acetonitrile on a Spheri-5 RP-18 cartridge column and a wavelength of 340 nm showed 60% enantiomeric excess of (S)-Pregabalin.

## Claims

1. A process for preparing a compound of Formula (VI), 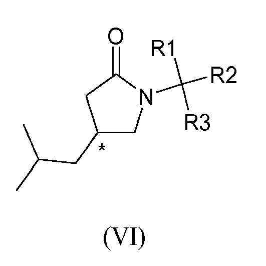 wherein:
R₁, R₂ and R₃ are each independently hydrogen, C₁-C₆ alkyl, C₃-C₆ cycloalkyl, aryl, heteroaryl, optionally substituted;
which comprises:
hydrogenating a compound of Formula (V), 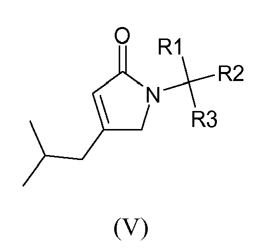 wherein R₁, R₂ and R₃ are as above defined,
in the presence of a catalyst.

2. The process according to claim 1, wherein the alkyl is methyl, ethyl or propyl; preferably methyl.

3. The process according to claim 1, wherein the aryl is phenyl, 1-naphtyl, or 2-naphtyl, optionally substituted; preferably phenyl.

4. The process according to claim 1, wherein the catalyst is an heterogeneous catalyst, an achiral or a chiral homogeneous catalyst.

5. The process according to claim 4, wherein the heterogeneous catalyst is a transition metal catalyst, such as Nickel, Copper, Palladium, Platinum, Rhodium, Ruthenium, Rhenium, Iridium or Gold, or mixtures thereof.

6. The process according to claim 5, wherein the heterogeneous catalyst is Palladium or Platinum.

7. The process according to claim 6, wherein the catalyst is Platinum.

8. The process according to claim 4, wherein the achiral homogeneous catalyst is a transition metal complex, such as (Ph₃P)₃RhCl, [(Ph₃P)₂Rh(COD)]PF₆; [(PCy₃)(Py)Ir(COD)]PF₆; or a catalyst prepared from transition metal salts, such as Rhodium, Ruthenium or Iridium salts, and achiral monodentate or bidentate ligands.

9. The process according to claim 8, wherein the catalyst is (Ph₃P)₃RhCl.

10. The process according to claim 4, wherein the chiral homogeneous catalyst is a chiral organometallic complex of transition metals salts, such as Rhodium, Ruthenium or Iridium salts, with an optically active ligand, such as atropisomeric biaryl bisphosphine ligands, chiral C₂ symmetric phosphorus ligands, phospholane ligands, chiral ferrocene-based bisphosphane based ligands, phosphinooxazolines, phosphinitooxazolines, phosphinitoimidazolines, or chiral phosphoramidite family Monophos.

11. The process according to claim 4, wherein the catalyst is in a supported form.

12. The process according to claim 11, wherein the support is charcoal.

13. The process according to claim 1, wherein the compound of Formula (VI) is 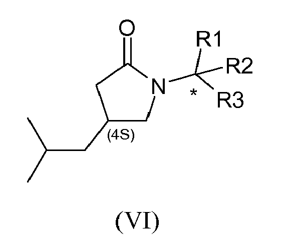 wherein R₁, R₂ and R₃ are as defined in claim 1.

14. The process according to claim 1, wherein said compound of Formula (V) is prepared by cyclization of a compound of Formula (IV) 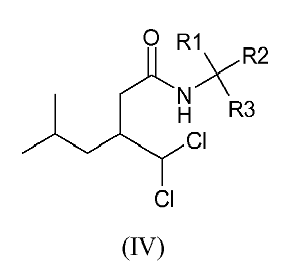 wherein R₁, R₂ and R₃ are as defined in claim 1.

15. The process according to claim 14, wherein the cyclization of a compound of Formula (IV) is carried out by heating in a suitable solvent, optionally in the presence of an halide salt.

16. The process according to claim 15, wherein the solvent is a lower alcohol.

17. The process according to claim 15, wherein the halide salt is lithium bromide or iodide; sodium, potassium, magnesium or calcium iodide; preferably potassium iodide.

18. The process according to claim 14, wherein said compound of Formula (IV) is prepared by reacting 2,2-dichloro-3-isobutylcyclobutanone of Formula (II) 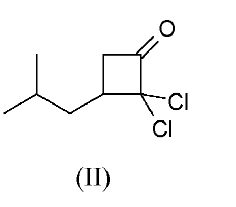 with a primary amine of Formula (III), 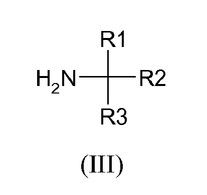 wherein R₁, R₂ and R₃ are as defined in claim 1.

19. The process according to claim 18, wherein the primary amine is methylamine, ethylamine, *n*-propylamine, *tert*-butylamine, benzylamine or (R)- or (S)-1-phenylethylamine.

20. The process according to claim 19, wherein the primary amine is benzylamine.

21. The process according to claim 19, wherein the primary amine is (S)-1-phenylethylamine.

22. The process according to claim 19, wherein the primary amine is (R)-1-phenylethylamine.

23. The process according to claim 18, wherein the reaction is carried out in an ether, preferably THF.

24. A compound of Formula (VI), or a hydrate, a solvate thereof, 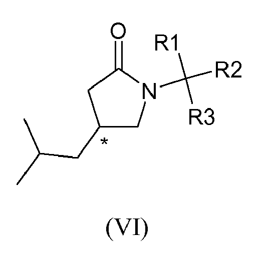 wherein:
R₁, R₂ and R₃ are each independently hydrogen, C₁-C₆ alkyl, C₃-C₆ cycloalkyl, aryl, heteroaryl, optionally substituted, with the proviso that said compound is not (4S)-isobutyl-1-((1S)-phenylethyl)-pyrrolidin-2-one, nor (4R)-isobutyl-1-((1S)-phenylethyl)-pyrrolidin-2-one.

25. A compound according to claim 24, wherein the alkyl group is methyl, ethyl or propyl; preferably methyl.

26. A compound according to claim 24, wherein the aryl is phenyl, 1-naphtyl, or 2-naphtyl, optionally substituted; preferably phenyl.

27. A compound according to claim 24, selected from:
(4S)-isobutyl-1-benzylpyrrolidin-2-one;
(4S)-isobutyl-1-((1R)-phenylethyl)-pyrrolidin-2-one;

28. A compound of Formula (V), or a hydrate, a solvate thereof, 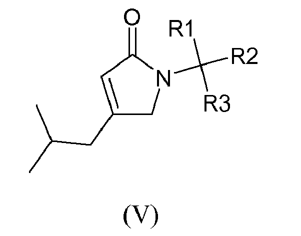 wherein:
R₁, R₂ and R₃ are each independently hydrogen, C₁-C₆ alkyl, C₃-C₆ cycloalkyl, aryl, heteroaryl, optionally substituted.

29. A compound according to claim 28, wherein the alkyl group is methyl, ethyl or propyl; preferably methyl.

30. A compound according to claim 28, wherein the aryl is phenyl, 1-naphtyl, or 2-naphtyl, optionally substituted; preferably phenyl.

31. A compound according to claim 28, selected from:
1-benzyl-4-isobutyl-1,5-dihydropyrrol-2-one
4-isobutyl-1-((1R)-phenylethyl)-1,5-dihydropyrrol-2-one
4-isobutyl-1-((1S)-phenylethyl)-1,5-dihydropyrrol-2-one

32. A compound of Formula (IV), or a hydrate, a solvate thereof, 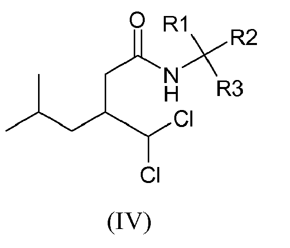 wherein:
R₁, R₂ and R₃ are each independently hydrogen, C₁-C₆ alkyl, C₃-C₆ cycloalkyl, aryl, heteroaryl, optionally substituted.

33. A compound according to claim 32, wherein the alkyl group is methyl, ethyl or propyl; preferably methyl.

34. A compound according to claim 32, wherein the aryl is phenyl, 1-naphtyl, or 2-naphtyl, optionally substituted; preferably phenyl.

35. A compound according to claim 32, selected from:
*N-*benzyl-3-dichloromethyl-5-methylhexanamide;
*N*-((1R)-phenylethyl)-3-dichloromethyl-5-methylhexanamide;
*N-*((1 S)-phenylethyl)-3-dichloromethyl-5-methylhexanamide.

36. A compound of Formula (II) or a hydrate, a solvate thereof,

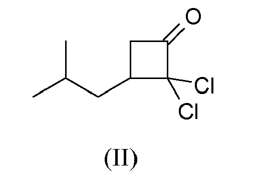

37. Use of compounds of Formula (II), (IV), (V) or (VI), as claimed in any one of claims 24-36, as intermediates in the preparation of Pregabalin.

38. A process for preparing Pregabalin, or a pharmaceutically acceptable salt, hydrate, solvate, or polymorph thereof, the process comprising:
hydrogenating a compound of Formula (V), 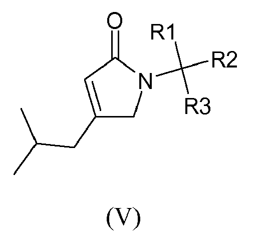
wherein R₁, R₂ and R₃ are as defined in claim 1;
in the presence of a catalyst, to give a compound of Formula (VI) 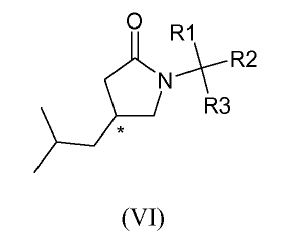 wherein R₁, R₂ and R₃ are as defined in claim 1.

39. The process according to claim 38, wherein the catalyst is an heterogeneous catalyst, an achiral or a chiral homogeneous catalyst.

40. The process according to claim 39, wherein the heterogeneous catalyst is a transition metal catalyst, such as Nickel, Copper, Palladium, Platinum, Rhodium, Ruthenium, Rhenium, Iridium or Gold, or mixtures thereof.

41. The process according to claim 40, wherein the heterogeneous catalyst is Palladium or Platinum.

42. The process according to claim 41, wherein the catalyst is Platinum.

43. The process according to claim 39, wherein the achiral homogeneous catalyst is a transition metal complex, such as (Ph₃P)₃RhCl, [(Ph₃P)₂Rh(COD)]PF₆; [(PCy₃)(Py)Ir(COD)]PF₆; or a catalyst prepared from transition metal salts, such as Rhodium, Ruthenium or Iridium salts, and achiral monodentate or bidentate ligands.

44. The process according to claim 43, wherein the catalyst is (Ph₃P)₃RhCl.

45. The process according to claim 39, wherein the chiral homogeneous catalyst is a chiral organometallic complex of transition metals salts, such as Rhodium, Ruthenium or Iridium salts, with an optically active ligand, such as atropisomeric biaryl bisphosphine ligands, chiral C₂ symmetric phosphorus ligands, phospholane ligands, chiral ferrocene-based bisphosphane based ligands, phosphinooxazolines, phosphinitooxazolines, phosphinitoimidazolines, or chiral phosphoramidite family Monophos.

46. The process according to claim 39, wherein the catalyst is in a supported form.

47. The process according to claim 46, wherein the support is charcoal.

48. The process according to claim 38, wherein the compound of Formula (VI) is

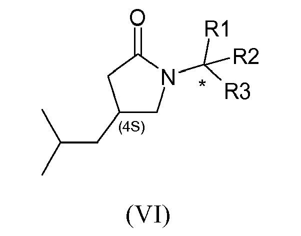

49. The process according to claim 38, wherein Pregabalin is (S)-Pregabalin.

50. The process according to claim 38, further comprising:
a) nitrogen deprotection of a compound of Formula (VI), to give a compound of Formula (VII) 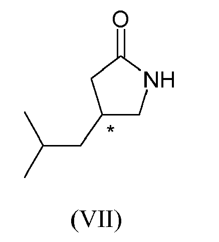 and hydrolyzing a compound of Formula (VII), to give Pregabalin;
or
b) hydrolysing or alcoholysing a compound of Formula (VI), to give a compound of Formula (VIII), 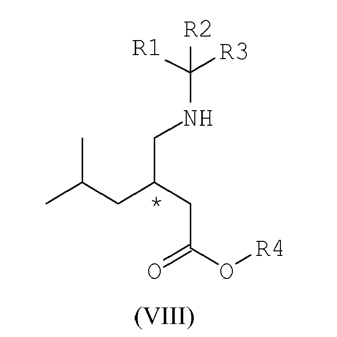
wherein R₁, R₂ and R₃ are as defined in claim 1 and R4 is an alkaline or earth alkaline cation, hydrogen, or C₁-C₆ alkyl, and following nitrogen deprotection of a compound of Formula (VIII), to give Pregabalin.

51. The process according to claim 50, step a), wherein the nitrogen deprotection of a compound of Formula (VI) to give a compound of Formula (VII), is carried out by treatment with a Bronsted acid, optionally in a suitable solvent.

52. The process according to claim 51, wherein the Bronsted acid is an inorganic or organic acid.

53. The process according to claim 52, wherein the acid is sulfuric acid or methanesulfonic acid.

54. The process according to claim 51, wherein the solvent is an organic solvent.

55. The process according to claim 54, wherein the solvent is toluene or xylenes.
